# EUROPEAN PATENT APPLICATION

(11) **EP 4 691 356 A1**
(43) Date of publication of application: **11.02.2026**
(21) Application number: 24807076.5
(22) Date of filing: 02.05.2024
(51) Int. Cl.: A61B 5/022

(54) **BLOOD PRESSURE METER, BLOOD PRESSURE MEASUREMENT METHOD, BLOOD PRESSURE MEASUREMENT PROGRAM, TRAINED MODEL CONSTRUCTION METHOD, AND TRAINED MODEL CONSTRUCTION PROGRAM**

(30) Priority: 12.05.2023 JP 2023079664
(71) Applicant: OMRON Corporation, Kyoto-shi, Kyoto 600-8530 (JP)
(72) Inventor: UCHIDA, Shihori, Kyoto-shi, Kyoto 600-8530 (JP); NISHIMOTO, Takashi, Kyoto-shi, Kyoto 600-8530 (JP)
(74) Representative: Isarpatent
(86) International application number: PCT/JP2024/016909
(87) International publication number: WO 2024/237123

(57) **Abstract**

The blood pressure meter includes: a cuff to be wrapped around an arm of a subject to be measured; a detection unit that detects cuff pressure; a decomposition unit that acquires, based on the cuff pressure, a first waveform indicating a time-series change in a feature value related to blood pressure of the subject to be measured and decomposes the first waveform into a plurality of second waveforms indicating two or more different feature values; a trained model constructed by machine learning in which the second waveforms for a plurality of test subjects are used as explanatory variables and blood pressure actually measured by auscultation is used as an objective variable; a measurement unit that measures the blood pressure of the subject by inputting the plurality of second waveforms into the trained model; and an output unit that outputs the blood pressure measured by the measurement unit.

## Description

### TECHNICAL FIELD

The present invention relates to a blood pressure meter, a blood pressure measurement method, a blood pressure measurement program, a trained model construction method, and a trained model construction program.

### BACKGROUND OF INVENTION

Blood pressure measurement is used to understand health conditions. In blood pressure measurement, attempts are being made to improve the accuracy of blood pressure measurements using machine learning, including deep learning. As an example of measuring blood pressure using deep learning, a method has been considered in which the shape of each pulse and the shape of the envelope of the pulse waveform are input into a model to measure blood pressure (see Non-patent Literature 1 to 3).

### CITATION LIST

### NON-PATENT LITERATURE

Non-patent Literature 1: Ahmadreza Argha, Branko G. Celler, Nigel H. Lovell, Artificial Intelligence Based Blood Pressure Estimation From Auscultatory and Oscillometric Waveforms: A Methodological Review, "IEEE REVIEWS IN BIOMEDICAL ENGINEERING", (USA), 2022, VOL. 15, p. 152-p. 168
Non-patent Literature 2: AHMADREZA ARGHA, JI WU, STEVEN W. SU, AND BRANKO G. CELLER, "Blood Pressure Estimation From Beat-by-Beat Time-Domain Features of Oscillometric Waveforms Using Deep-Neural-Network Classification Models," [online], August 6, 2019, IEEE, [retrieved March 28, 2023], Internet <URL: https://ieeexplore.ieee.org/document/8789404>
Non-patent Literature 3: Ahmed S. Alghamdi, Kemal Polat, Abdullah Alghoson, Abdulrahman A. Alshdadi, Ahmed A. Abd El-Latif, Gaussian process regression (GPR) based non-invasive continuous blood pressure prediction method from cuff oscillometric signals, [online], February 20, 2020, [retrieved March 28, 2023], Internet <URL:https://www.sciencedirect.com/science/article/abs/pii/S0003682X20301079?via%3Dihub >

### SUMMARY

### TECHNICAL PROBLEM

When measuring blood pressure using the oscillometric method, blood pressure is measured based on the waveform of the pulse wave, and therefore, if the pulse wave includes noise, the accuracy of the blood pressure measurement decreases. The noise included in the pulse wave can increase depending on the way of wrapping the cuff, the position where the cuff is wrapped, and the like. Therefore, in order to suppress the influence of the noise, calibration of the measured blood pressure and data collection for setting parameters of a blood pressure measurement algorithm are performed. However, even if calibration and parameter setting for the blood pressure measurement algorithm are performed, there is no guarantee that measurement errors due to noise can be removed, and the cost of collecting data is also required.

One aspect of the disclosed technology aims to provide a blood pressure meter, a blood pressure measurement method, a blood pressure measurement program, a trained model construction method, and a trained model construction program that can perform blood pressure measurement with as high accuracy as possible without performing data collection for setting parameters of a blood pressure measurement algorithm or calibration of a measurement result.

### SOLUTION TO PROBLEM

One aspect of the disclosed technology is exemplified by the following blood pressure meter. The blood pressure meter includes: a cuff to be wrapped around an arm of a subject to be measured; a detection unit that detects cuff pressure received by the cuff from the arm; a decomposition unit that acquires, based on the cuff pressure, a first waveform indicating a time-series change in a feature value related to blood pressure of the subject to be measured and decomposes the first waveform into a plurality of second waveforms indicating two or more different feature values; a trained model constructed by machine learning in which the second waveform for a test subject is used as an explanatory variable and blood pressure actually measured by auscultation is used as an objective variable; a measurement unit that measures the blood pressure of the subject to be measured by inputting the plurality of second waveforms into the trained model; and an output unit that outputs the blood pressure measured by the measurement unit.

According to the blood pressure meter, the first waveform indicating the time-series change in the feature value related to the blood pressure of the subject to be measured is decomposed into the plurality of second waveforms indicating two or more different feature values. That is, each of the second waveforms can be said to represent the first waveform from a plurality of viewpoints. Therefore, when the first waveform includes noise, it is conceivable that even if the noise has a significant influence on the second waveform relating to a certain feature value, the noise has a small influence on the second waveform relating to another feature value. Therefore, according to the blood pressure meter, it is possible to perform blood pressure measurement with as high accuracy as possible without performing data collection for setting parameters of a blood pressure measurement algorithm or calibration of a measurement result.

Here, in the blood pressure meter, the first waveform may include a cuff pressure waveform indicating a time-series change in the cuff pressure, the decomposition unit may decompose the cuff pressure waveform into a plurality of third waveforms indicating two or more different feature values, the trained model may be constructed using the third waveform for the test subject as the explanatory variable, and the measurement unit may measure the blood pressure of the subject to be measured by inputting the third waveform into the trained model.

In the blood pressure meter, the first waveform may include a pulse waveform indicating a time-series change in a pulse wave of the subject to be measured, the pulse waveform being acquired based on a time-series change in the cuff pressure, the decomposition unit may decompose the pulse waveform into a plurality of fourth waveforms indicating two or more different feature values, the trained model may be constructed using the fourth waveform for the test subject as the explanatory variable, and the measurement unit may measure the blood pressure of the subject to be measured by inputting the fourth waveform into the trained model.

In the blood pressure meter, the first waveform may include a cuff pressure waveform indicating a time-series change in the cuff pressure and a pulse waveform indicating a time-series change in a pulse wave of the subject to be measured, the pulse waveform being acquired based on the time-series change in the cuff pressure, the decomposition unit may decompose the cuff pressure waveform into a plurality of third waveforms indicating two or more different feature values, and decompose the pulse waveform into a fourth waveform obtained from the time-series change in the cuff pressure, the trained model may be constructed using the third waveform and the fourth waveform for the test subject as the explanatory variables, and the measurement unit may measure the blood pressure of the subject to be measured by inputting the third waveform and the fourth waveform into the trained model. The decomposition unit may include a Wave-U-Net.

The disclosed technology can also be understood from the aspects of a blood pressure measurement method, a blood pressure measurement program, a trained model construction method, and a trained model construction program.

### ADVANTAGEOUS EFFECTS OF INVENTION

According to the disclosed technology, it is possible to perform blood pressure measurement with as high accuracy as possible without performing data collection for setting parameters of a blood pressure measurement algorithm or calibration of a measurement result.

### BRIEF DESCRIPTION OF THE DRAWINGS

FIG. 1 is a diagram illustrating an example of a configuration of a blood pressure meter according to an embodiment.
FIG. 2 is a diagram schematically illustrating a state in which the blood pressure meter is worn on a wrist of a subject to be measured.
FIG. 3 is a diagram schematically illustrating processing by a waveform decomposition unit according to the embodiment.
FIG. 4 is a first diagram schematically illustrating processing by a blood pressure measurement unit according to the embodiment.
FIG. 5 is a second diagram schematically illustrating processing by the blood pressure measurement unit according to the embodiment.
FIG. 6 is a first diagram schematically illustrating the construction of a blood pressure model according to the embodiment.
FIG. 7 is a second diagram schematically illustrating the construction of the blood pressure model according to the embodiment.
FIG. 8 is a third diagram schematically illustrating the construction of the blood pressure model according to the embodiment.
FIG. 9 is a fourth diagram schematically illustrating the construction of the blood pressure model according to the embodiment.
FIG. 10 is a fifth diagram schematically illustrating the construction of the blood pressure model according to the embodiment.
FIG. 11 is a diagram illustrating an example of a processing flow of the blood pressure meter according to the embodiment.
FIG. 12 is a diagram illustrating a verification result of the accuracy of blood pressure measurement by the blood pressure meter according to the embodiment.

### DESCRIPTION OF EMBODIMENTS

### <Application Example>

An application example of the present invention will be described. A blood pressure meter 100 according to an application example is a device that measures the blood pressure of a subject to be measured by wrapping a cuff 120 around the upper arm or wrist of the subject to be measured. In the blood pressure meter 100, a measurement unit 114 inputs at least one of the time-series change in the cuff pressure and the time-series change in the pulse wave to a blood pressure model 117, and acquires the blood pressure of the subject to be measured from the blood pressure model 117.

The measurement unit 114 controls the pressure of the cuff 120 and acquires, from the pressure sensor 113, a time-series change in a feature value related to the blood pressure of the subject to be measured. Examples of the time-series change in the feature value related to the blood pressure of the subject to be measured include a time-series change in the cuff pressure and a time-series changes in the waveform of the Oscillometric Waveform (OMW).

The measurement unit 114 includes a waveform decomposition unit 115 and a blood pressure measurement unit 116. The waveform decomposition unit 115 decomposes the time-series change in the feature value related to the blood pressure of the subject to be measured into a plurality of second waveforms (mode waveforms). The mode waveform includes a waveform obtained by decomposing the time-series change in the feature value related to the blood pressure of the subject to be measured based on waveform features such as high and low frequency and large and small amplitude. The mode waveform may include, for example, a waveform that globally indicates an entirety of the time-series change in the feature value related to the blood pressure of the subject to be measured, or a waveform that locally indicates a portion of the time-series change in the feature value related to the blood pressure of the subject to be measured. The waveform decomposition unit 115 includes, for example, Wave-U-Net.

The blood pressure measurement unit 116 inputs the plurality of mode waveforms decomposed by the waveform decomposition unit 115 to the blood pressure model 117 and acquires the blood pressure of the subject to be measured from the blood pressure model 117. The blood pressure measurement unit 116 outputs the acquired blood pressure to the output unit 118.

The blood pressure model 117 is a trained model that outputs the blood pressure of the subject to be measured when a plurality of mode waveforms are input. The blood pressure model 117 is constructed by machine learning in which a plurality of mode waveforms obtained by decomposing the time-series change in the feature value related to the blood pressure of the subject to be measured are used as explanatory variables and blood pressure measured by auscultation is used as an objective variable. Machine learning includes deep learning.

According to this application example, the mode waveform includes a waveform that indicates a global change in the time-series change of the feature value related to the blood pressure of the subject to be measured, and a waveform that indicates a local change. Therefore, the blood pressure model 117 can learn both the global feature and the local feature of the time-series change in the feature value related to the blood pressure of the subject to be measured in an even manner. According to such a blood pressure model 117, even if noise is included in the time-series change in the feature value related to the blood pressure of the subject to be measured, the blood pressure of the subject to be measured can be output with high accuracy. That is, according to this application example, even if noise is included in the time-series change in the feature value related to the blood pressure of the subject to be measured, which is acquired by the measurement unit 114, highly accurate blood pressure measurement can be achieved without performing data collection for setting parameters of the blood pressure measurement algorithm or calibration of the measurement result.

### <Embodiment>

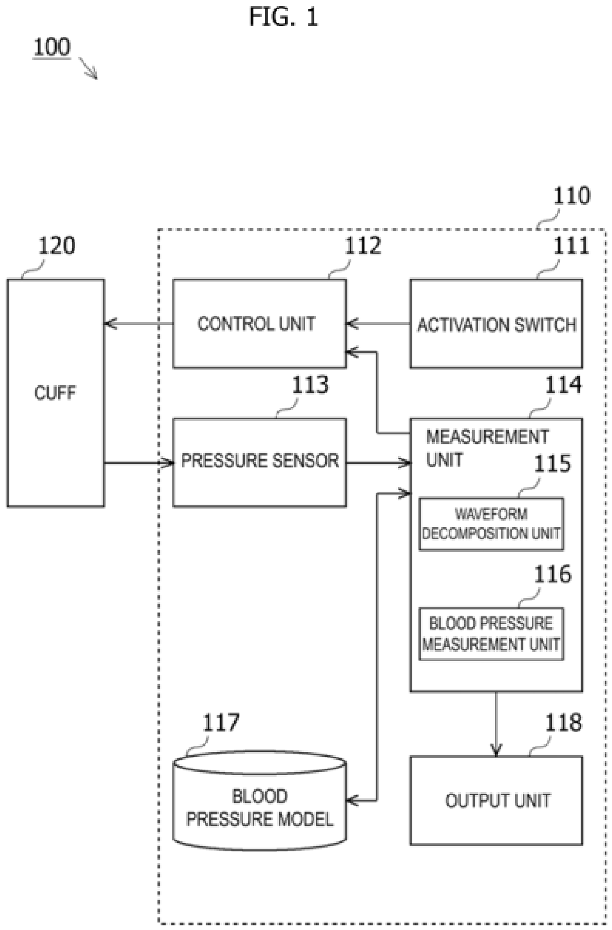

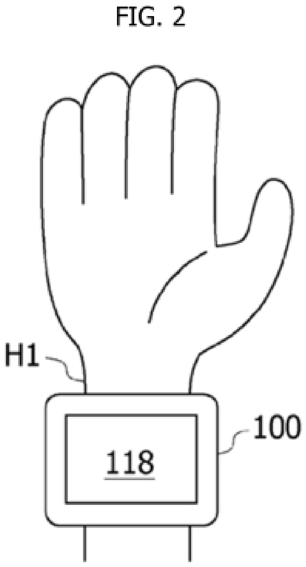

An embodiment will be described below with reference to the drawings. FIG. 1 is a diagram illustrating an example of a configuration of a blood pressure meter 100 according to an embodiment. FIG. 2 is a diagram schematically illustrating a state in which the blood pressure meter 100 is worn on a wrist H1 of a subject to be measured. The blood pressure meter 100 includes a main body 110 and a cuff 120. The blood pressure meter 100 measures the blood pressure of a subject to be measured by wrapping the cuff 120 around, for example, the wrist H1 of the subject to be measured. The position where the cuff 120 is wrapped is not limited to the wrist H1, but may be an upper arm of the subject to be measured.

The cuff 120 is formed in a band shape and has a bag inside which air is supplied. When air is supplied to the bag inside the cuff 120 while the cuff 120 is wrapped around the upper arm or the like of the subject to be measured, the pressure applied by the cuff to the upper arm or the like increases. Furthermore, when air is inhaled from the bag inside the cuff 120 while the cuff 120 is wrapped around the upper arm or the like of the subject to be measured, the pressure applied by the cuff to the upper arm or the like decreases.

The main body 110 is equipped with, for example, a processor and a storage unit, and the processor executes a program stored in the storage unit to realize each processing unit such as an activation switch 111, a control unit 112, a pressure sensor 113, a measurement unit 114, a blood pressure model 117, and an output unit 118. The measurement unit 114 includes a waveform decomposition unit 115 and a blood pressure measurement unit 116. In other words, the blood pressure meter 100 can be considered as a computer having a cuff 120. Each processing unit such as the activation switch 111, the control unit 112, the pressure sensor 113, the measurement unit 114, the blood pressure model 117, and the output unit 118 may be a dedicated hardware circuit. In the blood pressure meter 100, when the activation switch 111 is turned on, the blood pressure meter 100 is activated and starts measuring blood pressure.

The control unit 112 controls the pressure applied by the cuff 120 to the upper arm or the like of the subject to be measured by supplying air to the cuff 120 and inhaling air from the cuff 120. When the activation switch 111 is turned on, the control unit 112 starts controlling the pressure of the cuff 120.

The pressure sensor 113 is a sensor that detects the pressure (cuff pressure) caused by the cuff 120. The pressure sensor 113 outputs the detected cuff pressure to the measurement unit 114. The cuff pressure includes, for example, the pressure applied by the cuff 120 to the upper arm or the like, and pressure vibrations caused by pulsation of blood flowing in the artery of the upper arm or the like.

The measurement unit 114 measures blood pressure based on the pressure detected by the pressure sensor 113. The time-series change in the cuff pressure of the cuff 120, which is increased to a pressure higher than the systolic blood pressure and then gradually reduced to a pressure lower than the diastolic blood pressure, is acquired from the pressure sensor 113. Furthermore, the measurement unit 114 extracts a time-series change in the pressure vibration caused by pulsation of blood flowing in the artery of the blood flow from the time-series change in the cuff pressure acquired from the pressure sensor 113. The waveform indicating the extracted time-series change in the cuff pressure (hereinafter also referred to as a cuff pressure waveform) and the waveform indicating the time-series change in the pressure vibration (hereinafter also referred to as a pulse waveform) are processed by the waveform decomposition unit 115 and the blood pressure measurement unit 116, respectively.

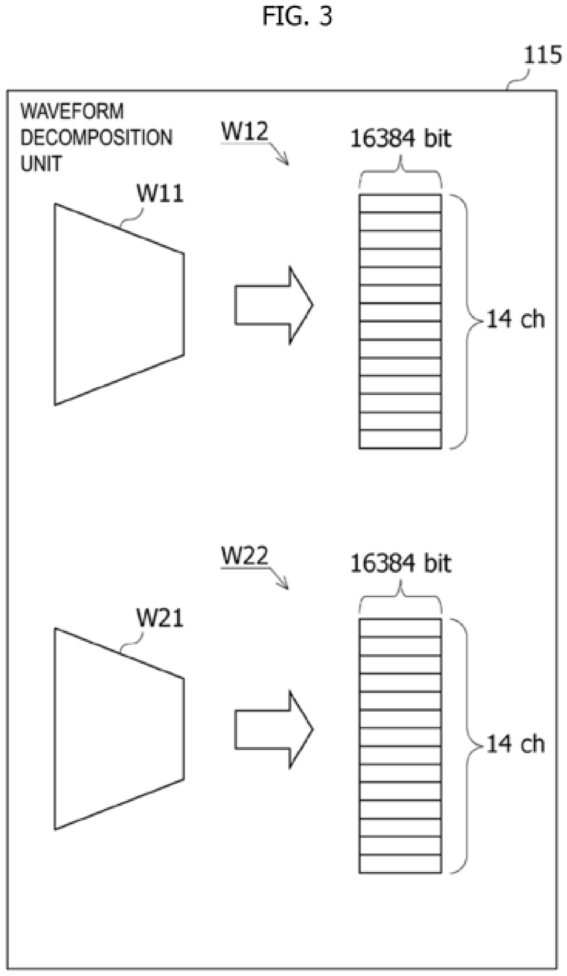

The waveform decomposition unit 115 decomposes each of the cuff pressure waveform and the pulse waveform into a plurality of mode waveforms. FIG. 3 is a diagram schematically illustrating the processing by the waveform decomposition unit 115 according to the embodiment. The waveform decomposition unit 115 decomposes, for example, a cuff pressure waveform W11 into mode waveforms of 14 channels, each with a data length of 16384 bits, to generate a mode waveform group W12. The waveform decomposition unit 115 also decomposes a pulse waveform W21 into mode waveforms of 14 channels, each with a data length of 16384 bits, to generate a mode waveform group W22.

The waveform decomposition unit 115 includes, for example, Wave-U-Net. Wave-U-Net is a trained model used, for example, for decomposing speech waveforms. Speech includes a non-periodic component in addition to a periodic component, and it is considered that the cuff pressure waveform and the pulse waveform also include a non-periodic component in addition to a periodic component, similarly to the speech. Therefore, in the present embodiment, it is assumed that the waveform decomposition unit 115 that decomposes the cuff pressure waveform and pulse waveform includes Wave-U-Net. In addition, the waveform decomposition unit 115 may include, for example, any one of DEMUCS, D3NET, Conv-TasNet, Wavenet, Sams-Net, and Meta-TasNet instead of Wave-U-Net.

Here, as described in the application example, mode waveform may include a waveform obtained by decomposing the time-series change in the feature value related to the blood pressure of the subject to be measured based on waveform features such as high and low frequency and large and small amplitude. The mode waveform may also include a waveform that globally indicates an entirety of the time-series change in the feature value related to the blood pressure of the subject to be measured, or a waveform that locally indicates a portion of the time-series change in the feature value related to the blood pressure of the subject to be measured. An example of a globally indicated waveform includes an envelope of a waveform that indicates a time-series change in a feature value related to blood pressure of a subject to be measured. An example of a locally indicated waveform includes a waveform obtained by extracting (pulling out) a portion of a waveform that indicates a time-series change in a feature value related to blood pressure of a subject to be measured.

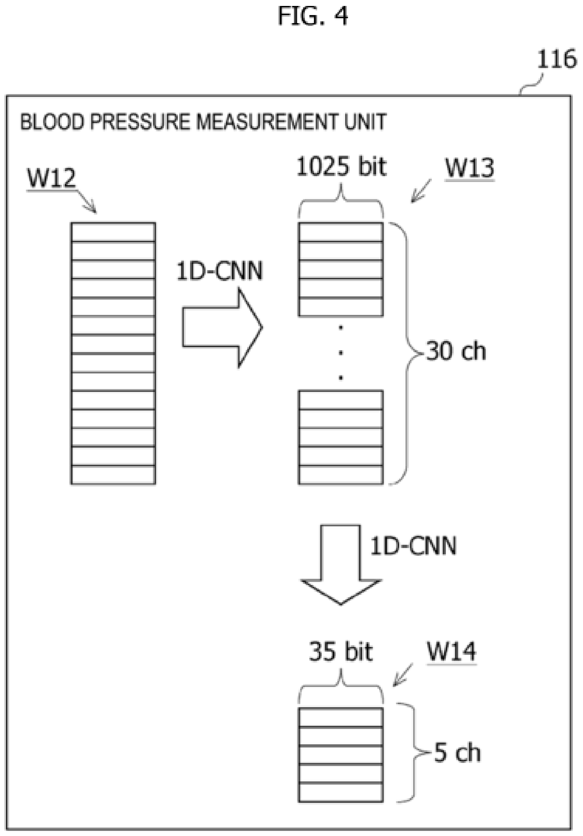

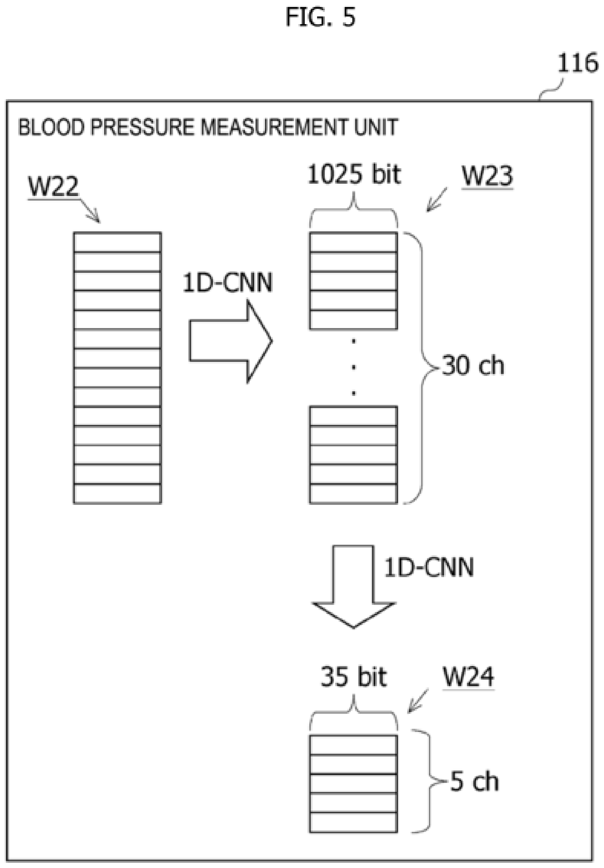

The blood pressure measurement unit 116 measures the blood pressure of the subject to be measured based on the mode waveform decomposed by the waveform decomposition unit 115. FIGS. 4 and 5 are diagrams schematically illustrating processing by the blood pressure measurement unit 116 according to the embodiment. The blood pressure measurement unit 116 applies a one-dimensional convolutional neural network (1D-CNN) to the mode waveform group W12 input from the waveform decomposition unit 115, and replaces the mode waveform group W12 with an intermediate feature value W13 of 30 channels, each with a data length of 1025 bits. The blood pressure measurement unit 116 further applies 1D-CNN to the intermediate feature value W3, and replaces the intermediate feature value W13 with an intermediate feature value W14 of five channels, each with a data length of 35 bits.

Furthermore, the blood pressure measurement unit 116 applies a one-dimensional convolutional neural network (1D-CNN) to a mode waveform group W22 input from the waveform decomposition unit 115, and replaces the mode waveform group W22 with an intermediate feature value W23 of 30 channels, each with a data length of 1025 bits. The blood pressure measurement unit 116 further applies 1D-CNN to the intermediate feature value W23, and replaces the intermediate feature value W23 with an intermediate feature value W24 of five channels, each with a data length of 35 bits. The blood pressure measurement unit 116 inputs the intermediate feature value W14 and the intermediate feature value W24 to the blood pressure measurement unit 116, and acquires the blood pressure of the subject to be measured as an output of the blood pressure measurement unit 116.

The blood pressure model 117 is a trained model that outputs blood pressure when the intermediate feature value W14 generated from the cuff pressure waveform and the intermediate feature value W24 generated from the pulse waveform are input. In other words, the blood pressure model 117 is a trained model that outputs blood pressure when the feature value of the cuff pressure waveform and the feature value of the pulse waveform are input. The blood pressure model 117 is stored in, for example, a storage unit accommodated in the main body 110.

The output unit 118 outputs the blood pressure measured by the blood pressure measurement unit 116. The output unit 118 is, for example, a liquid crystal display (LCD), a plasma display panel (PDP), an inorganic electroluminescence (EL) panel, or an organic EL panel. The output unit 118 may be a printer or a speaker.

### <Construction of Blood Pressure Model 117>

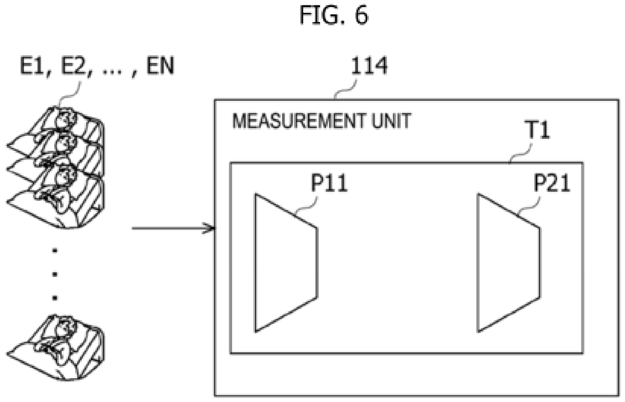

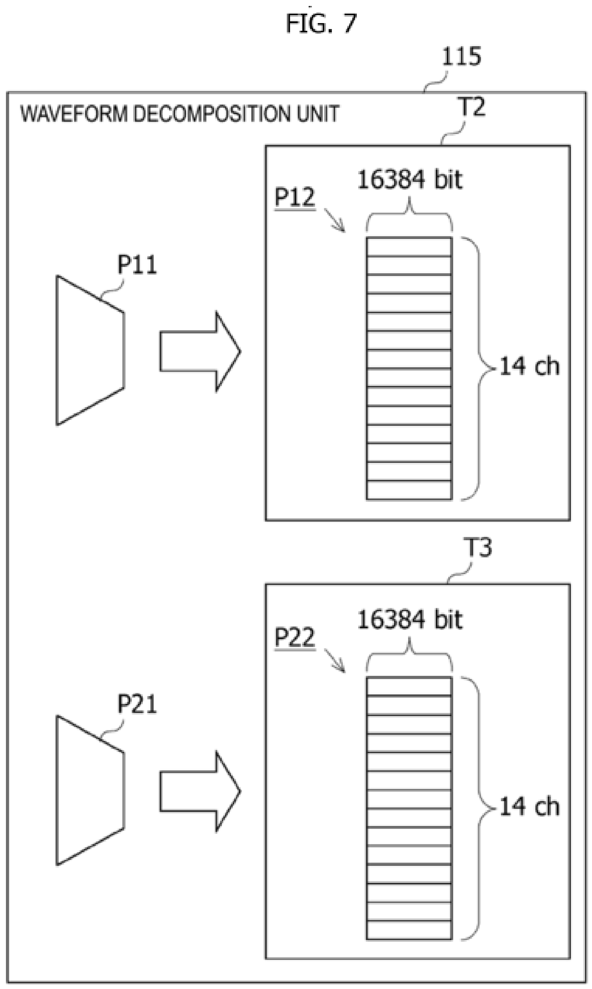

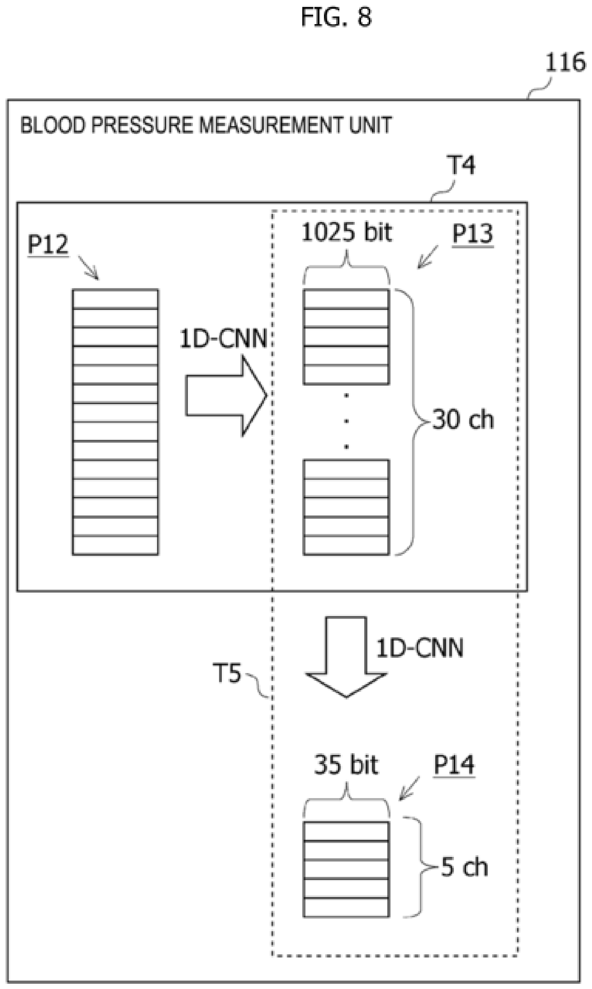

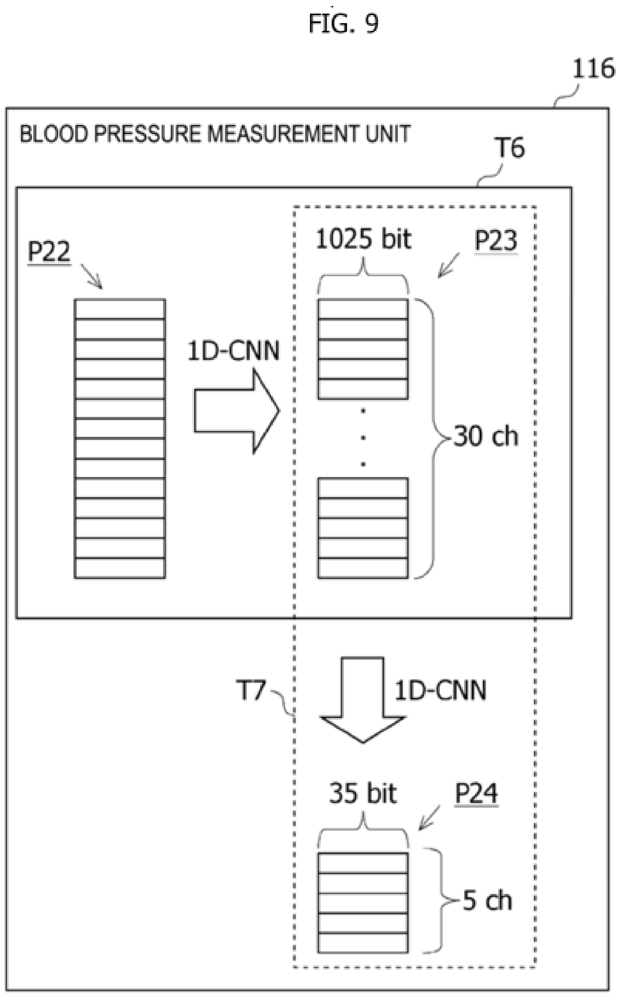

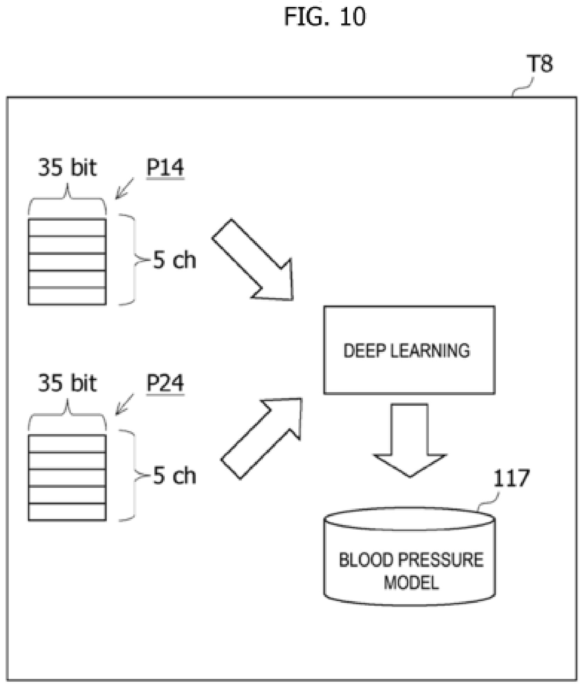

FIGS. 6 to 10 are diagrams schematically illustrating the construction of the blood pressure model 117 according to the embodiment. The construction of the blood pressure model 117 will be described with reference to FIGS. 6 to 10. In constructing the blood pressure model 117, a plurality of test subjects (E1, E2, ..., EN) are prepared. In the present embodiment, a plurality of test subjects are prepared, but the number of test subjects prepared may be one. In step T1, the measurement unit 114 acquires a cuff pressure waveform P11 and a pulse waveform P21 of a test subject E1.

In step T2, the waveform decomposition unit 115 decomposes the cuff pressure waveform P11 into mode waveforms of 14 channels, each with a data length of 16384 bits, to generate a mode waveform group P12. In step T3, the waveform decomposition unit 115 decomposes the pulse waveform P21 into mode waveforms of 14 channels, each with a data length of 16384 bits, to generate a mode waveform group P22.

In step T4, the blood pressure measurement unit 116 applies 1D-CNN to the mode waveform group P12 input from the waveform decomposition unit 115, and replaces the mode waveform group P12 with an intermediate feature value P13 of 30 channels, each with a data length of 1025 bits. In step T5, the blood pressure measurement unit 116 applies 1D-CNN to the intermediate feature value P13, and replaces the intermediate feature value W23 with an intermediate feature value P14 of five channels, each with a data length of 35 bits.

In step T6, the blood pressure measurement unit 116 applies 1D-CNN to the mode waveform group P22 input from the waveform decomposition unit 115, and replaces the mode waveform group P22 with an intermediate feature value P23 of 30 channels, each with a data length of 1025 bits. In step T7, the blood pressure measurement unit 116 applies 1D-CNN to the intermediate feature value P23, and replaces the intermediate feature value P23 with an intermediate feature value P24 of five channels, each with a data length of 35 bits.

In step T8, a blood pressure model 117 is generated by machine learning in which the intermediate feature value P14 generated from the cuff pressure waveform and the intermediate feature value P24 generated from the pulse waveform are used as explanatory variables and blood pressure is used as the objective variable. The blood pressure used as the objective variable is, for example, blood pressure measured by auscultation. The processes from steps T1 to T8 are also performed for each of test subjects E2 to EN.

### <Processing Flow>

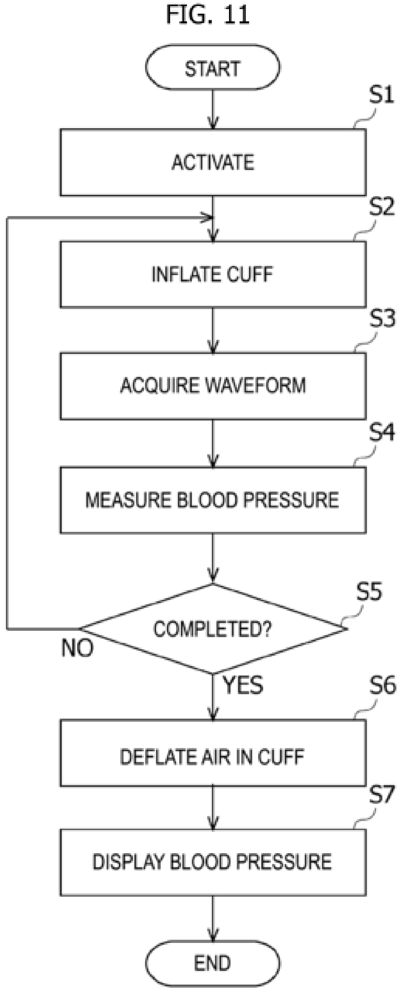

FIG. 11 is a diagram illustrating an example of a processing flow of the blood pressure meter 100 according to the embodiment. Hereinafter, an example of a processing flow of the blood pressure meter 100 will be described with reference to FIG. 4.

In step S1, the activation switch 111 is turned on, thereby activating the blood pressure meter 100. In step S2, the control unit 112 supplies air to the cuff 120 to pressurize the wrist of the subject to be measured. The control unit 112 inflates the cuff 120 to a pressure higher than the systolic blood pressure, and then gradually reduces the pressure in the cuff 120.

In step S3, the measurement unit 114 acquires a cuff pressure waveform and a pulse waveform based on the cuff pressure detected by the pressure sensor 113. Furthermore, the waveform decomposition unit 115 decomposes the waveform acquired in step S3 into a plurality of mode waveforms. In step S4, the blood pressure measurement unit 116 measures the blood pressure of the subject to be measured using the blood pressure model 117 based on the mode waveform decomposed in step S3.

In step S5, the measurement unit 114 determines whether the measurement of the blood pressure of the subject to be measured has been completed. For example, the measurement unit 114 may determine that blood pressure measurement has been completed when both the systolic blood pressure and the diastolic blood pressure of the subject to be measured have been measured. If completed (YES in step S5), the process proceeds to step S6. If not completed (NO in step S5), the process proceeds to step S2.

In step S6, the control unit 112 deflates the air in the cuff 120. In step S7, the output unit 118 outputs the blood pressure measured in step S4.

### <Verification>

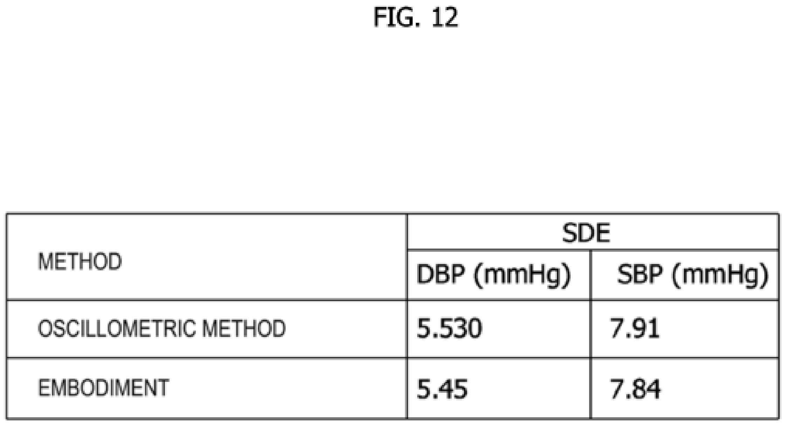

The accuracy of blood pressure measurement by the blood pressure meter 100 according to the embodiment described above has been verified, and will be described below. FIG. 12 is a diagram illustrating a verification result of the accuracy of blood pressure measurement by the blood pressure meter 100 according to the embodiment. In FIG. 12, the blood pressure measured by the blood pressure meter 100 and the blood pressure measured by the oscillometric method are compared.

In this verification, blood pressure was measured a plurality of times for each of a plurality of test subjects and measurement results were acquired. In this verification, the acquired measurement results are evaluated based on Standard Deviation of Error (SDE). With reference to FIG. 12, the "embodiment" showed higher accuracy than the "oscillometric method" for both the diastolic blood pressure: DBP and the systolic blood pressure: SBP.

### <Advantageous Effects of Embodiment>

In the present example, each of the cuff pressure waveform and the pulse waveform is decomposed into a plurality of mode waveforms. The mode waveform includes a waveform that indicates a global change in the time-series change of the feature value related to the blood pressure of the subject to be measured, and a waveform that indicates a local change. Therefore, the blood pressure model 117 can learn both the global feature and the local feature of the time-series change in the feature value related to the blood pressure of the subject to be measured in an even manner. According to such a blood pressure model 117, even if noise is included in the cuff pressure waveform or the pulse waveform, the blood pressure of the subject to be measured can be output with high accuracy. That is, according to the present embodiment, even if noise is included in the cuff pressure waveform or the pulse waveform due to the influence of the position at which the cuff 120 is wrapped or the posture of the subject to be measured, highly accurate blood pressure measurement can be achieved without performing calibration or parameter setting of the blood pressure measurement algorithm.

### <Modification Example>

In the embodiment described above, both the cuff pressure waveform and the pulse waveform are input to the waveform decomposition unit 115, but either the cuff pressure waveform or the pulse waveform may be input to the waveform decomposition unit 115. In this case, the blood pressure model 117 only needs to be constructed by using, as an explanatory variable, the intermediate feature value generated from the waveform used for blood pressure measurement, out of the intermediate feature value generated from the cuff pressure waveform and the intermediate feature value generated from the pulse waveform.

The embodiment and modification example disclosed above can be combined.

### <Computer-Readable Recording Medium>

An information processing program for causing a computer or any other machine or device (hereinafter referred to as a computer or the like) to implement any of the functions described above may be recorded in a recording medium readable by the computer or the like. The functions can be provided by causing the computer or the like to read and execute the program from the recording medium.

Here, the term "computer-readable recording medium" refers to a recording medium that stores information such as data or programs by electrical, magnetic, optical, mechanical, or chemical means, and that can be read by a computer or the like. Examples of such a recording medium that is removable from the computer or the like include a flexible disk, a magnetooptical disk, a compact disc read-only memory (CD-ROM), a compact disc-recordable (CD-R), a compact disc-rewritable (CD-RW), a digital versatile disc (DVD), a Blu-ray disc (BD), a digital audio tape (DAT), a 8mm tape, a flash memory, an external hard disk drive, and a solid state drive (SSD). Examples of a recording medium fixed to the computer or the like include a built-in type hard disk drive, an SSD, and a ROM.

### <Supplementary Note 1>

A blood pressure meter (100) including:
a cuff (120) to be wrapped around an arm (H1) of a subject to be measured;
a detection unit (113) that detects cuff pressure received by the cuff (120) from the arm (H1);
   a decomposition unit (115) that acquires, based on the cuff pressure, a first waveform (W11, W21) indicating a time-series change in a feature value related to blood pressure of the subject to be measured and decomposes the first waveform (W11, W21) into a plurality of second waveforms (W12, W22) indicating two or more different feature values;
   a trained model (117) constructed by machine learning in which the second waveform (W12, W22) for a test subject is used as an explanatory variable and blood pressure actually measured by auscultation is used as an objective variable;
   a measurement unit (116) that measures the blood pressure of the subject to be measured by inputting the plurality of second waveforms (W12, W22) into the trained model; and
   an output unit (118) that outputs the blood pressure measured by the measurement unit.

### <Supplementary Note 2>

The blood pressure meter (100) according to Supplementary Note 1, in which
the first waveform (W11, W21) includes a cuff pressure waveform (W11) indicating a time-series change in the cuff pressure,
   the decomposition unit (115) decomposes the cuff pressure waveform (W11) into a plurality of third waveforms (W12) indicating two or more different feature values,
the trained model (117) is constructed using the third waveform (P14) for the test subject (E1, ..., EN) as the explanatory variable, and
   the measurement unit (116) measures the blood pressure of the subject to be measured by inputting the third waveform (W12) into the trained model (117).

### <Supplementary Note 3>

The blood pressure meter (100) according to Supplementary Note 1 or 2, in which
the first waveform (W11, W21) includes a pulse waveform (W21) indicating a time-series change in a pulse wave of the subject to be measured, the pulse waveform being acquired based on a time-series change in the cuff pressure,
the decomposition unit (115) decomposes the pulse waveform (W21) into a plurality of fourth waveforms (W22) indicating two or more different feature values,
the trained model (117) is constructed using the fourth waveform (W24) for the test subject (E1, ..., EN) as the explanatory variable, and
the measurement unit (116) measures the blood pressure of the subject to be measured by inputting the fourth waveform (W22) into the trained model (117).

### <Supplementary Note 4>

The blood pressure meter (100) according to any one of Supplementary Notes 1 to 3, in which
the first waveform (W11, W21) includes a cuff pressure waveform (W11) indicating a time-series change in the cuff pressure and a pulse waveform (W21) indicating a time-series change in a pulse wave of the subject to be measured, the pulse waveform being acquired based on the time-series change in the cuff pressure,
the decomposition unit (115) decomposes the cuff pressure waveform (W11) into a plurality of third waveforms (W12) indicating two or more different feature values, and decomposes the pulse waveform (W21) into a fourth waveform (W22) obtained from the time-series change in the cuff pressure,
the trained model (117) is constructed using the third waveform (P14) and the fourth waveform (P24) for the test subject (E1, ..., EN) as the explanatory variables, and
the measurement unit (116) measures the blood pressure of the subject to be measured by inputting the third waveform (W12) and the fourth waveform (W22) into the trained model (117).

### <Supplementary Note 5>

The blood pressure meter according to any one of Supplementary Notes 1 to 4, in which the decomposition unit (115) includes a Wave-U-Net.

### <Supplementary Note 6>

A blood pressure measurement method including:
detecting, by a computer, cuff pressure received by a cuff (120) to be wrapped around an arm (H1) of a subject to be measured from the arm (H1);
acquiring, by the computer, based on the cuff pressure, a first waveform (W11, W21) indicating a time-series change in a feature value related to blood pressure of the subject to be measured and decomposing the first waveform (W11, W21) into a plurality of second waveforms (W12, W22) indicating two or more different feature values;
measuring, by the computer, the blood pressure of the subject to be measured by inputting the plurality of second waveforms (W12, W22) into a trained model (117) constructed by machine learning in which the second waveform (W12, W22) for a test subject (E1, ..., EN) is used as an explanatory variable and blood pressure actually measured by auscultation is used as an objective variable; and
outputting, by the computer, the blood pressure measured in the measuring.

### <Supplementary Note 7>

A blood pressure measurement program for causing a computer to execute:
detecting cuff pressure received by a cuff (120) to be wrapped around an arm (H1) of a test subject (E1, ..., EN) from the arm (H1);
acquiring, based on the cuff pressure, a first waveform (W11, W21) indicating a time-series change in a feature value related to blood pressure of the subject to be measured and decomposing the first waveform (W11, W21) into a plurality of second waveforms (W12, W22) indicating two or more different feature values;
measuring the blood pressure of the subject to be measured by inputting the plurality of second waveforms (W12, W22) into a trained model (117) constructed by machine learning in which the second waveform (W12, W22) is used as an explanatory variable and blood pressure actually measured by auscultation is used as an objective variable; and
outputting the blood pressure measured in the measuring.

### <Supplementary Note 8>

A trained model construction method including:
detecting, by a computer, cuff pressure received by a cuff (120) to be wrapped around an arm (H1) of a test subject (E1, ..., EN) from the arm (H1);
acquiring, by the computer, based on the cuff pressure, a first waveform (W11, W21) indicating a time-series change in a feature value related to blood pressure of the subject to be measured and decomposing the first waveform (W11, W21) into a plurality of second waveforms (W12, W22) indicating two or more different feature values; and
constructing, by the computer, a trained model (117) by machine learning in which the second waveform (W12, W22) is used as an explanatory variable and blood pressure actually measured by auscultation is used as an objective variable.

### <Supplementary Note 9>

A trained model construction program for causing a computer to execute:
detecting cuff pressure received by a cuff (120) to be wrapped around an arm (H1) of a test subject from the arm (H1);
acquiring, based on the cuff pressure, a first waveform indicating a time-series change in a feature value related to blood pressure of the subject to be measured and decomposing the first waveform (W11, W21) into a plurality of second waveforms (W12, W22) indicating two or more different feature values; and
constructing a trained model (117) by machine learning in which the second waveform (W12, W22) is used as an explanatory variable and blood pressure actually measured by auscultation is used as an objective variable.

### REFERENCE SIGNS

100: Blood pressure meter
110: Main body
111: Activation switch
112: Control unit
113: Pressure sensor
114: Measurement unit
115: Waveform decomposition unit
116: Blood pressure measurement unit
117: Blood pressure model
118: Output unit
120: Cuff
H1: Wrist

## Claims

1. A blood pressure meter comprising:
a cuff to be wrapped around an arm of a subject to be measured;
a detection unit that detects cuff pressure received by the cuff from the arm;
a decomposition unit that acquires, based on the cuff pressure, a first waveform indicating a time-series change in a feature value related to blood pressure of the subject to be measured and decomposes the first waveform into a plurality of second waveforms indicating two or more different feature values;
a trained model constructed by machine learning in which the second waveform for a test subject is used as an explanatory variable and blood pressure actually measured by auscultation is used as an objective variable;
a measurement unit that measures the blood pressure of the subject to be measured by inputting the plurality of second waveforms into the trained model; and
an output unit that outputs the blood pressure measured by the measurement unit.

2. The blood pressure meter according to claim 1, wherein
the first waveform includes a cuff pressure waveform indicating a time-series change in the cuff pressure,
the decomposition unit decomposes the cuff pressure waveform into a plurality of third waveforms indicating two or more different feature values,
the trained model is constructed using the third waveform for the test subject as the explanatory variable, and
the measurement unit measures the blood pressure of the subject to be measured by inputting the third waveform into the trained model.

3. The blood pressure meter according to claim 1, wherein
the first waveform includes a pulse waveform indicating a time-series change in a pulse wave of the subject to be measured, the pulse waveform being acquired based on a time-series change in the cuff pressure,
the decomposition unit decomposes the pulse waveform into a plurality of fourth waveforms indicating two or more different feature values,
the trained model is constructed using the fourth waveform for the test subject as the explanatory variable, and
the measurement unit measures the blood pressure of the subject to be measured by inputting the fourth waveform into the trained model.

4. The blood pressure meter according to claim 1, wherein
the first waveform includes a cuff pressure waveform indicating a time-series change in the cuff pressure and a pulse waveform indicating a time-series change in a pulse wave of the subject to be measured, the pulse waveform being acquired based on the time-series change in the cuff pressure,
the decomposition unit decomposes the cuff pressure waveform into a plurality of third waveforms indicating two or more different feature values, and decomposes the pulse waveform into a fourth waveform obtained from the time-series change in the cuff pressure,
the trained model is constructed using the third waveform and the fourth waveform for the test subject as the explanatory variables, and
the measurement unit measures the blood pressure of the subject to be measured by inputting the third waveform and the fourth waveform into the trained model.

5. The blood pressure meter according to any one of claims 1 to 4, wherein the decomposition unit includes a Wave-U-Net.

6. A blood pressure measurement method comprising:
detecting, by a computer, cuff pressure received by a cuff to be wrapped around an arm of a subject to be measured from the arm;
acquiring, by the computer, based on the cuff pressure, a first waveform indicating a time-series change in a feature value related to blood pressure of the subject to be measured and decomposing the first waveform into a plurality of second waveforms indicating two or more different feature values;
measuring, by the computer, the blood pressure of the subject to be measured by inputting the plurality of second waveforms into a trained model constructed by machine learning in which the second waveform for a test subject is used as an explanatory variable and blood pressure actually measured by auscultation is used as an objective variable; and
outputting, by the computer, the blood pressure measured in the measuring.

7. A blood pressure measurement program for causing a computer to execute:
detecting cuff pressure received by a cuff to be wrapped around an arm of a test subject from the arm;
acquiring, based on the cuff pressure, a first waveform indicating a time-series change in a feature value related to blood pressure of the subject to be measured and decomposing the first waveform into a plurality of second waveforms indicating two or more different feature values;
measuring the blood pressure of the subject to be measured by inputting the plurality of second waveforms into a trained model constructed by machine learning in which the second waveform is used as an explanatory variable and blood pressure actually measured by auscultation is used as an objective variable; and
outputting the blood pressure measured in the measuring.

8. A trained model construction method comprising:
detecting, by a computer, cuff pressure received by a cuff to be wrapped around an arm of a test subject from the arm;
acquiring, by the computer, based on the cuff pressure, a first waveform indicating a time-series change in a feature value related to blood pressure of the subject to be measured and decomposing the first waveform into a plurality of second waveforms indicating two or more different feature values; and
constructing, by the computer, a trained model by machine learning in which the second waveform is used as an explanatory variable and blood pressure actually measured by auscultation is used as an objective variable.

9. A trained model construction program for causing a computer to execute:
detecting cuff pressure received by a cuff to be wrapped around an arm of a test subject from the arm;
acquiring, based on the cuff pressure, a first waveform indicating a time-series change in a feature value related to blood pressure of the subject to be measured and decomposing the first waveform into a plurality of second waveforms indicating two or more different feature values; and
constructing a trained model by machine learning in which the second waveform is used as an explanatory variable and blood pressure actually measured by auscultation is used as an objective variable.
